(19) Europäisches Patentamt
European Patent Office
Office européen des brevets

(11) **EP 4 748 387 A1**

(12) **EUROPEAN PATENT APPLICATION**

(43) Date of publication:
**27.05.2026 Bulletin 2026/22**

(21) Application number: **25217061.8**

(22) Date of filing: **19.11.2025**

(51) International Patent Classification (IPC):
*A61K 36/537* (2006.01)   *A61K 36/886* (2006.01)
*A61K 38/48* (2006.01)   *A61P 1/04* (2006.01)

(52) Cooperative Patent Classification (CPC):
(C-Sets available)
**A61K 38/4873; A61K 36/537; A61K 36/886;**
**A61P 1/04**   (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **22.11.2024 IT 202400026367**

(71) Applicant: **Camasco di Carlo Maria, Scornajenghi**
**87100 Cosenza (IT)**

(72) Inventors:
• **Scornajenghi, Carlo Maria**
**87100 COSENZA (IT)**
• **Sinicropi, Maria Stefania**
**87036 Rende (CS) (IT)**

• **Gabriele, Domenico**
**87036 Rende (CS) (IT)**
• **Veneziano, Alessandro**
**87036 Rende (CS) (IT)**
• **Vattimo, Anna Francesca**
**87010 Terranova da Sibari (CS) (IT)**
• **Esposito, Pasquale**
**75025 Policoro (MT) (IT)**
• **Brogno, Maria Assunta**
**87040 Luzzi (CS) (IT)**
• **Perna, Pilerio**
**87067 Cosenza (IT)**

(74) Representative: **Rigamonti, Dorotea et al**
**THINX S.r.l.**
**Piazzale Luigi Cadorna, 10**
**20123 Milano (IT)**

(54) **COMPOSITION COMPRISING ALOE VERA, BROMELAIN, AND SAGE, AND ITS USES**

(57) The present invention relates to a composition comprising Aloe vera, Bromelain and Sage for use as a supplement in the treatment of mucosal lesions of the esophageal tract and/or for the treatment of symptoms of gastroesophageal reflux.

FIG. 1

EP 4 748 387 A1

(52) Cooperative Patent Classification (CPC): (Cont.)

C-Sets
**A61K 36/537, A61K 2300/00;**
**A61K 36/886, A61K 2300/00;**
**A61K 38/4873, A61K 2300/00**

**Description**

**TECHNICAL FIELD**

The present invention refers to a composition comprising Aloe vera, Bromelain and Sage. In one embodiment, said composition is for use as a supplement in the preventive and/or curative treatment of gastroesophageal reflux disease (GERD) and heartburn associated therewith.

**BACKGROUND**

Gastroesophageal reflux disease (GERD) is a clinical problem caused by pathological complications of the gastroesophageal reflux. It can be referred to as "disease" when reflux causes symptoms or when inflammatory lesions of the esophagus, ulcers or metaplastic transformation of the mucosa are evidenced with gastroscopy.

Reflux is primarily due to relaxation of the lower esophageal sphincter (LES), which may be due to insufficient LES pressure with respect to the increase in abdominal pressure or progressive weakening of the closure pressure.

The pathophysiology of GERD is characterized by a multifactorial nature and is associated with an imbalance between the harmful effects of reflux and an ineffective defence system, which may or may not be accompanied by a defective valve mechanism at the esophagogastric junction.

GERD is a chronic, recurrent and progressive disease associated with a wide range of esophageal complications, such as esophageal ulcer, esophageal cancer, Barrett's disease and non-esophageal complications, such as respiratory problems, chest pain, angina. Mucosal damage caused by gastric reflux causes GERD to occasionally present with symptoms similar to functional dyspepsia and irritable bowel syndrome.

Epidemiological data have consistently demonstrated an increase in the prevalence of GERD worldwide. The worldwide frequency of the disease is estimated to be between 8% and 33%.

In the last century, GERD has gone from being a rare diagnosis to one of the most frequent diagnoses in clinical practice among Western populations. Some risk factors are becoming more prevalent in the population, such as smoking, the use of non-steroidal anti-inflammatory drugs, and obesity. Despite this, the well-established increase in its prevalence is also explained by concrete advances in the understanding of the characteristics of the disease and in the diagnostic methods. One of the major advances has been the recognition that a clear correlation between the severity of reflux-induced symptoms and the presence of endoscopically evident erosion is not observed for most patients. Considering this distinction, GERD can be classified based on the presence or absence of erosions, defining non-erosive reflux disease (NERD) and erosive esophagitis (EE).

The most common symptom of GERD is a retrosternal burning sensation called pyrosis. It is estimated that 20-40% of patients who complain of pyrosis will, in fact, receive a diagnosis of GERD. This symptom is associated with prostaglandin E2 (PGE2) production in patients with GERD, but the mechanism of production is unclear. Some studies have shown that PGE2 production increases following exposure to weak acids via TRPV 4 /ERK/cPLA 2 in esophageal epithelial cells, suggesting a role in the symptoms of GERD.

The conditions resulting from erosive reflux disease include reflux esophagitis, reflux stenosis, Barrett's esophagus, and esophageal adenocarcinoma. In contrast, the non-erosive reflux disease is linked to disorders where the patient reports chest pain, heartburn, or regurgitation. However, there is no sign of damage to the esophageal mucosa. In addition to the conditions that have a suggested relationship with the disease (such as inflammation of the pharynx, sinusitis, idiopathic pulmonary fibrosis, and recurrent otitis media), the extraesophageal disorders linked to heartburn include disorders that are known to be associated with GERD, such as tooth damage, inflammation of the larynx, cough, and asthma.

The treatment for gastric reflux disease includes medications, lifestyle changes, and long-term surgical alternatives. Proton pump inhibitors (PPIs), more effective than histamine receptor antagonists ($H_2RA$), are the mainstay of the medical therapy for acidity control.

Antacids and alginate-based medications can also be used to relieve symptoms. However, in some patients, these treatments may be ineffective and cause side effects, such as intestinal disorders (diarrhoea, flatulence, abdominal pain), rashes, palpitations and, in case of prolonged use, an increased risk of osteoporosis and bone fragility.

At present, the problem of developing a treatment or prevention for the symptoms of gastroesophageal reflux and GERD that overcomes the limits of previous techniques, in particular with regard to side effects, has not yet been solved.

The problem of developing a solution that is able to exert an anti-inflammatory action and favour the process of re-epithelialization and wound healing of mucosal lesions in the esophageal tract, caused by the rise of acidic and/or basic vapours from the stomach, quickly and effectively, also remains unsolved. Therefore, there is still a need to develop compositions for the treatment of gastroesophageal reflux that are well tolerated, effective and free of side effects, especially for patients who need to take anti-reflux drugs for long periods.

The medicinal herbs exhibiting antioxidant and anti-inflammatory properties show efficacy in the treatment of GERD. Basim Al-Sulivany, et al. in Pathology And Medicinal Plant Treatment Of Gastroesophageal Reflux Disease. African J Gastroenterology and Hepatology vol 7. 2024, describe the effect of Curcuma longa and Morus alba on the reduction of gastric acid. The potential benefit of the medicinal plants over traditional antisecretory drugs lies in their ability to treat NERDs, an area where PPIs show no possible impact.

US 2011/038945 and US 2009/208588 describe the use of liquid compositions comprising honey for the treatment of GERD.

IT201800004332A1 describes the use of honey in combination with Aloe vera and hyaluronic acid for the treatment of GERD.

Aloe vera is used in the treatment of gastroesophageal reflux and heartburn for its soothing, anti-inflammatory and regenerating properties.

Bromelain, an enzyme extracted from pineapple, is used in the treatment of gastroesophageal reflux and heartburn for its anti-inflammatory and digestive properties. In the context of GERD, bromelain can help reduce inflammation of the esophageal mucous membranes and improve digestion, promoting a faster breakdown of the proteins in the digestive tract. This digestive effect can reduce the pressure in the stomach, thus decreasing the risk of acid reflux.

The Sage (*Salvia officinalis*) extract is used in the treatment of GERD and heartburn for its anti-inflammatory, antioxidant and astringent properties. Sage is known to aid digestion and soothe irritations of the mucous membranes of the gastrointestinal tract, helping to reduce symptoms of acid reflux and irritation of the esophagus. In addition, thanks to its astringent properties, Sage can help tone the mucosal tissues, making them less susceptible to irritation caused by acid reflux.

The need to have alternative methods to those in use, effective and free of side effects, remains strongly felt.

**DESCRIPTION**

A composition based on natural substances is proposed, capable of treating, preventing and/or alleviating the symptoms linked to gastroesophageal reflux and correlated disease, with the advantage of being practically free of the side effects associated with conventional treatments.

**DESCRIPTION OF THE FIGURES**

**Figure 1:** Anti-inflammatory action.

**Figure 2:** Cell viability.

**Figure 3:** Evaluation of the TEER of the inserts treated for 1 h with the test samples and subsequently subjected to acid insult for 15'.

**Figure 4:** Evaluation of the TEER of the inserts treated for 1 h with the test samples and subsequently subjected to acid insult for 45'.

**Figure 5:** Evaluation of the Lucifer Yellow Flux of the inserts treated for 1 h with the test samples and subsequently subjected to acid insult for 15'.

**Figure 6:** Evaluation of the Lucifer Yellow Flux of the inserts treated for 1 h with the test samples and subsequently subjected to acid insult for 45'.

**Figure 7:** IL-6 concentration determined in the culture media of the inserts treated for 24 h with the test samples after 1' acid insult.

**Figure 8:** Exemplary images of the morphological observations made on the inserts treated for 24h with the indicated samples after 1-minute acid insult.

**Figure 9:** Exemplary images of the Wound healing assay on the MCF-10A breast epithelial cell line.

**DETAILED DESCRIPTION**

The present invention relates to a composition comprising Aloe vera, Bromelain and Sage.

In one embodiment, a whole-leaf extract of said Aloe vera is used.

In one embodiment, a leaf extract of said Sage is used.

The composition described here performs an anti-inflammatory, antioxidant, protective action and favours the re-epithelialization and wound healing of mucosal lesions of the esophageal tract, without inducing the side effects typical of the conventional treatments.

In one embodiment, said composition comprises, per 100 g of finished product:

| Aloe vera extract (A1) | 60-80 % |
| --- | --- |
| Sage Extract (S1) | 15-25 % |
| Bromelain (B1) | 0.5-2 % |
| Water | q.s. |

In one embodiment, said composition comprises, per 100 g of finished product:

| Aloe vera extract (A1) | 70 % |
| --- | --- |
| Sage Extract (S1) | 20 % |
| Bromelain (B1) | 1 % |
| Water | q.s. |

Aloe vera extract A1 was produced using a Naviglio extractor according to the following percentages:

| Water | q.s. 100 |
| --- | --- |
| Aloe Vera whole leaves | 30 - 60 % |

Sage extract S1 was produced using a Naviglio extractor according to the following percentages:

| Water | q.s. 100 |
| --- | --- |
| Sage leaves | 10 - 30 % |

In one embodiment, said composition also comprises technological additives and/or food- or pharmaceutical-grade excipients.

By way of example, said excipients are selected from the group comprising: diluents, adsorbents, lubricants, glidants, colorants, surfactants, antioxidants, sweeteners, flavorings, binders, disaggregants, plasticizers, viscosifiers, emulsifiers, humectants, wetting agents, preservatives, chelators and the like.

Said composition is formulated in liquid, solid, semi-solid form, preferably in liquid form.

Further object of the present invention is said composition for use in preventing, treating and/or alleviating the symptoms of gastroesophageal reflux.

In one embodiment, the administration of the composition to the subject takes place orally, for example in the form of a solution, suspension or syrup, or of a tablet, capsule, food comprising it or in any other form known to the person skilled in the art.

Further object of the present invention is a supplement comprising Aloe vera, Bromelain and Sage for use in the prevention and improvement of mucosal lesions of the esophageal tract and/or in the prevention and improvement of the symptoms of gastroesophageal reflux.

Below are some examples of the present invention, to be understood as non-limiting. The scope of the invention is defined by the following claims.

**EXAMPLES**

## Example 1: preparation of compositions according to the present invention

The following liquid formulations have been prepared:

Formulation 1

| Water | Q.s. 100 |
|---|---|
| Aloe vera extract A1 | 70 % |
| Bromelain B1 | 1 % |
| Sage extract S1 | 20 % |
| Xanthan gum | 0.1 - 1% |
| Sodium hyaluronate | 0.1 - 1 % |
| Flavoring | 0.1 - 1 % |
| Potassium sorbate | 0.01 - 1 % |
| Sodium benzoate | 0.01 - 1 % |

Formulation 2

| Water | Q.s. 100 |
|---|---|
| Aloe vera extract A1 | 70 % |
| Bromelain B1 | 1 % |
| Sage extract S1 | 20 % |
| Carboxymethylcellulose | 0.1-1% |
| Gum arabic | 0.1 - 1 % |
| Pectin | 0.1 - 1 % |
| Flavoring | 0.1 -1 % |
| Potassium sorbate | 0.01-1 % |
| Sodium benzoate | 0.01-1 % |

Formulation 3

| Water | Q.s. 100 |
|---|---|
| Aloe vera extract A1 | 70 % |
| Sage extract S1 | 20 % |
| Bromelain B1 | 1 % |
| Soya lecithin | 0.1 - 1 % |
| Pectin | 0.1 - 1% |
| Calcium carbonate | 0.1 - 1 % |
| Flavoring | 0.1 - 1 % |

(continued)

| Potassium sorbate | 0.01-1 % |
|---|---|
| Sodium benzoate | 0.01-1 % |

## Example 2: preparation of compositions for comparative purposes

For comparative purposes, the following formulations have been prepared:

Aloe vera extract A2

| Water | q.s. 100 |
|---|---|
| Aloe Vera Extract | 70 % |

Sage extract S2

| Water | q.s. 100 |
|---|---|
| Sage extract | 20 % |

Bromelain B2

| Water | q.s. 100 |
|---|---|
| Bromelain | 1% |

Control saline solution)

| Water | q.s. 100 |
|---|---|
| NaCl | 0.9% |

## Example 3: mucoadhesion test

Formulations 1, 2 and 3 according to the present invention and the comparative compositions as per Example 2 were subjected to in vitro Mucoadhesion Test on buccal mucosal cells.

In particular, mucoadhesiveness is determined by evaluating the percentage of inhibition of lectin-glycoprotein binding. Buccal mucosal cells are treated with biotinylated lectin (Con-A) which has a high affinity for glycosidic and mannosidic residues present in membrane glycoproteins. The cells are then exposed to streptavidin peroxidase, washed and the protein-glucose-lectin-biotinastreptavidin-peroxidase complex is quantified, thanks to the presence of peroxidase, by the ortho-phenylenediamine oxidation reaction.

The intensity of the yellow-orange staining of the solution, measured with a spectrophotometer at $\lambda$=450 nm, is proportional to the amount of glycoprotein-lectin bonds and thus to the amount of available sites (glycoproteins) for mucoadhesion.

In determining the mucoadhesiveness of a product, the cells are pretreated with the test sample and, if the test product contains mucoadhesive substances, these will bind to the glycosidic and mannosidic sites present in the membrane glycoproteins. In the subsequent step, with the addition of the biotinylated lectin, streptavidin peroxidase and ortho-phenylenediamine sequence, a staining is obtained that is less intense than that of the control, since a part of the glucosidic sites available for binding with the Con-A have already been engaged by the mucoadhesive substances present in the test product.

The decrease in the absorbance value is proportional to the ability of the test substances to "mucoadhere" to the mucosal cells.

The mucoadhesive capacity is expressed as a percentage of inhibition of the glycoprotein-lectin bond, or better, as a percentage of the mucoadhesion of the product, according to the equation:

$$\text{Product mucoadhesion percentage} = (1 - \text{abs sample}/\text{abs control}) \times 100$$

The oral cavity of donors (aged 24-27 years), who had neither eaten nor drunk for at least 60 minutes, was gently scraped with a wooden spatula and the cells thus harvested immersed in 0.05 M TBS (Tris Buffer Saline) pH 7.6.

After counting with Trypan blue 0.5%, the cells were diluted with NaCl 0.9% until a final concentration of 200,000 cells was obtained for each sample to be used in the experimentation. The cells were then left at a temperature of 4 °C.

The cell suspensions were then centrifuged at 2000 rpm for 5 minutes and incubated with 5 ml of a solution obtained by diluting each sample in saline solution in order to obtain a 1:4 dilution. For the control, the cell suspensions were instead incubated with 5 ml of 0.9% NaCl.

The incubation was continued for 60 minutes at 37 °C with gentle shaking.

After 3 washes with TBS, the buccal cells were incubated at 37 °C for 60 minutes with 5 ml of a solution containing Con-A at a concentration of 50 $\mu$g/ml; after a further 3 washes with TBS, they were incubated at 37 °C for 30 minutes with 5 ml of streptavidin peroxidase.

After 3 washes with TBS, 2.5 ml of o-phenylenediamine (o-pd) in 0.05 M citrate phosphate and $H_2O_2$ were added to the cells and, after 5 minutes, the reaction was stopped with $H_2SO_4$ 1M. The absorbance values for the individual determinations were then read using a spectrophotometer.

The individual samples were repeated 3 times in duplicate and the results reported represent the means $\pm$ SEM of all the experiments carried out.

Table 1

| % mucoadhesiveness | Interpretation |
| --- | --- |
| 0-20 % | SATISFACTORY |
| 20-40 % | FAIR |
| 40-80 % | GOOD |
| 80-100 % | EXCELLENT |

The results obtained, reported in Table 2, demonstrate a good mucoadhesiveness of the synergistic composition according to the invention, with a marked increase compared to that observed when the individual components are tested individually.

Table 2

| Sample | Abs $\lambda$=450nm | | | | Mucoadhesiveness (%) |
| --- | --- | --- | --- | --- | --- |
| | I | II | III | Average | |
| Control | 1.67 | 1.68 | 1.87 | 1.740 | 0.00% |
| Aloe extract (A2) | 1.11 | 0.99 | 1.13 | 1.07 | 36.66% |
| Sage extract (S2) | 1.73 | 1.80 | 1.77 | 1.766 | 1.49% |
| Bromelain (B2) | 1.75 | 1.79 | 1.78 | 1.773 | 1.92% |
| Formulation 1 | 0.60 | 0.57 | 0.59 | 0.586 | 65.49% |
| Formulation 2 | 0.61 | 0.59 | 0.58 | 0.593 | 65.92% |
| Formulation 3 | 0.56 | 0.59 | 0.59 | 0.58 | 65.88% |

## Example 4: Evaluation of antioxidant activity - Scavenger activity on the DPPH radical

DPPH (2,2'-diphenyl-1-picrylhydrazyl) is an organic free radical with a maximum absorption peak at 515-528 nm and allows the antioxidant activity to be evaluated by means of a spectrophotometric type measurement. The reaction involves the reduction of the DPPH radical by the molecules under examination, when antioxidants, with the formation of a yellow compound, diphenylpicrylhydrazine. The extent of the reduction reaction depends on the ability of the molecules to act as electron donors.

In order to evaluate the antioxidant properties, the scavenger activity against DPPH radicals was determined by adding the composition under analysis to 9.9 ml of a DPPH (200 $\mu$M) solution prepared in ethanol. The absorbance was read after 15 min at 517 nm. The experiment was also conducted simultaneously on a control sample, prepared in the absence of a sample and containing an equivalent amount of distilled water. The scavenger activity was calculated according to equation (1):

$$\% \, Inhibition = \frac{A_0 - A_1}{A_0} \times 100$$

$$(1)$$

wherein $A_0$ represents the absorbance of the control and $A_1$ is the absorbance of the analysed sample.

The results were expressed as IC50, i.e. the concentration of extract in $\mu$g/mL capable of bringing about a 50% inhibition of the DPPH radical. Trolox was used as a positive control (Table 3).

The result was compared with the data obtained following treatment with the individual components of the synergistic composition.

## Example 5: Evaluation of antioxidant activity - Scavenger activity on the ABTS radical

ABTS (2,2'-azinobis-(3-ethylbenzothiazoline-6-sulfonic acid)) is a radical of a hydrophilic nature that has an absorption maximum around 734 nm. Also in this case, the reaction depends on the ability of the antioxidant to donate hydrogen atoms. The scavenger action on the ABTS radical was studied by measuring the absorbance of the radical cation (ABTS • +). This method is based on the ability of the antioxidants to act on the radical cation ABTS, a blue-green chromophore with a characteristic absorption at 734 nm. ABTS • + was generated by the reaction of an aqueous solution of ABTS (7 mM) with 2.45 mM potassium persulfate ($K_2S_2O_8$). The obtained mixture was left in the dark at room temperature and, after 16 hours, it was diluted with distilled water to an absorbance of $0.999 \pm 0.030$ at 734 nm.

To evaluate the antioxidant properties of the product, an aliquot was added to 1 ml of the final ABTS solution and to 0.9 ml of distilled water. A control was prepared under the same reaction conditions but using distilled water in place of the sample. After 6 minutes, the absorbance was measured at 734 nm.

The scavenger activity was calculated according to equation (1):

$$\% \, Inhibition = \frac{A_0 - A_1}{A_0} \times 100$$

$$(1)$$

wherein $A_0$ represents the absorbance of the control and $A_1$ is the absorbance of the analysed sample.

The results were expressed as IC50, i.e. the concentration of extract in $\mu$g/mL capable of bringing about a 50% inhibition of the ABTS radical. Trolox was used as a positive control Table 3).

Table 3

|  | DPPH IC 50 $\mu$g/mL | ABTS IC 50 $\mu$g/mL |
|---|---|---|
| Aloe vera extract A2 | 817 $\mu$g/mL | 681 $\mu$g/mL |
| Sage extract S2 | 392 $\mu$g/mL | 273 $\mu$g/ mL |
| Bromelain B2 | 3624 $\mu$g/ mL | 3391 $\mu$g/ mL |
| Formulation 1 | 289 $\mu$g/ mL | 195 $\mu$g/mL |
| Formulation 2 | 284 $\mu$g/ mL | 197 $\mu$g/ mL |

(continued)

|  | DPPH IC 50 μg/mL | ABTS IC 50 μg/mL |
|---|---|---|
| Formulation 3 | 285 μg/ mL | 194 μg/ mL |
| Trolox | 6.50 μg/mL | 4.97 μg/ mL |

As is evident from the data reported in Table 3, the composition according to the invention showed a better scavenger capacity towards the radical ABTS and DPPH compared to the individual components of the composition. The IC50 values, i.e. the extract concentration capable of bringing about a 50% inhibition of the DPPH radical, were found to be 289 μg/mL for formulation 1, 284 μg/mL for formulation 2 and 285 μg/mL for formulation 3, while the IC50 values calculated for the antiradical activity against ABTS were found to be 195 μg/mL for formulation 1, 197 μg/mL for formulation 2 and 194 μg/mL for formulation 3.

These experiments, as well as those reported in Example 3, clearly show that the synergistic effect is attributable to the three extracts combined with each other, and is not impacted by the excipients in which the three extracts are formulated.

### Example 6: Anti-inflammatory activity evaluation

Cell viability assay

Cell viability was assayed by MTT test. The assay consists of using cells that are deprived of serum for 24 h, in such a way as to allow their synchronization at the same phase of the cell cycle. Subsequently, they are treated with the test samples for 72h at different concentrations, in triplicate, at the end of which the MTT is added to the final concentration of 0.5 mg/mL and the cells are left to incubate for about 2 hours at 37 °C. After the incubation time, the medium containing the MTT is aspirated from the wells and the DMSO (dimethyl sulfoxide) is added in order to solubilize the violet crystals that have formed inside the cells. The formation of these crystals occurs because MTT (3-(4,5-dimethylthiazol-2-yl)-2,5-diphenyltetrazolium bromide), i.e. a yellow tetrazolium salt, is reduced by the viable cells into formazan crystals by mitochondrial reductases. Thus only the viable cells can convert MTT to formazan and the amount of crystals formed is directly proportional to the number of viable cells. In contrast, the non-viable cells will not be able to convert MTT to formazan salts. The intensity of the staining, after solubilization of the crystals with DMSO, is measured using the spectrophotometer at a wavelength of 570 nm and then the cell viability will be calculated as a percentage of the absorbance obtained for the various extracts with respect to the absorbance of the control. The control represents nothing more than cells treated with the only vehicle in which the test extracts are dissolved (DMSO).

Scavenger activity on the nitric oxide

Using the Griess assay, using the RAW 264.7 murine macrophages, it was possible to study the anti-inflammatory activity of the composition. This is possible because following an inflammatory stimulus, the macrophages produce nitric oxide (NO) and pro-inflammatory cytokines such as TNF-a, IL-6. Overproduction of these mediators is a marker of numerous inflammatory diseases. In particular, in the 48-well multi-wells, 80,000 cells (RAW 264.7) were plated, then incubated for 24 h at 37 °C. After 24 h, the treatments are performed by simultaneously adding the samples of interest and the lipopolysaccharide (LPS) to the final concentration of 1 μg/mL in phenol red-free medium containing 1% FBS. LPS induces inflammation as it stimulates the production of NO in the cells of the immune system such as the macrophages.

After 24 h, 100 μL of medium present in the 48-well MW are mixed with 100 μL of Griess reagent in a 96-well MW (1:1 ratio). This reagent allows the quantification of the NO released by the macrophages in the treatment medium. This is a colorimetric assay with formation of a pink staining that is directly proportional to the amount of NO in the medium. It is allowed to stir for 30 minutes and, subsequently, the absorbance is read on the spectrophotometer at a wavelength of 540 nm. The absorbance obtained was then compared to that of the positive control, in which the cells were treated with the vehicle (DMSO) and LPS. In parallel, the MTT assay was performed under the same experimental conditions and at the same concentrations in order to verify the effect of the emulgels on the viability of the RAW 264.7 cells.

The preparations of Aloe vera extract (A2), Sage extract (S2) and bromelain (B2) indicated in Example 2 and the compositions described in Example 1 were used at the concentrations indicated (μg/mL) in the figures.

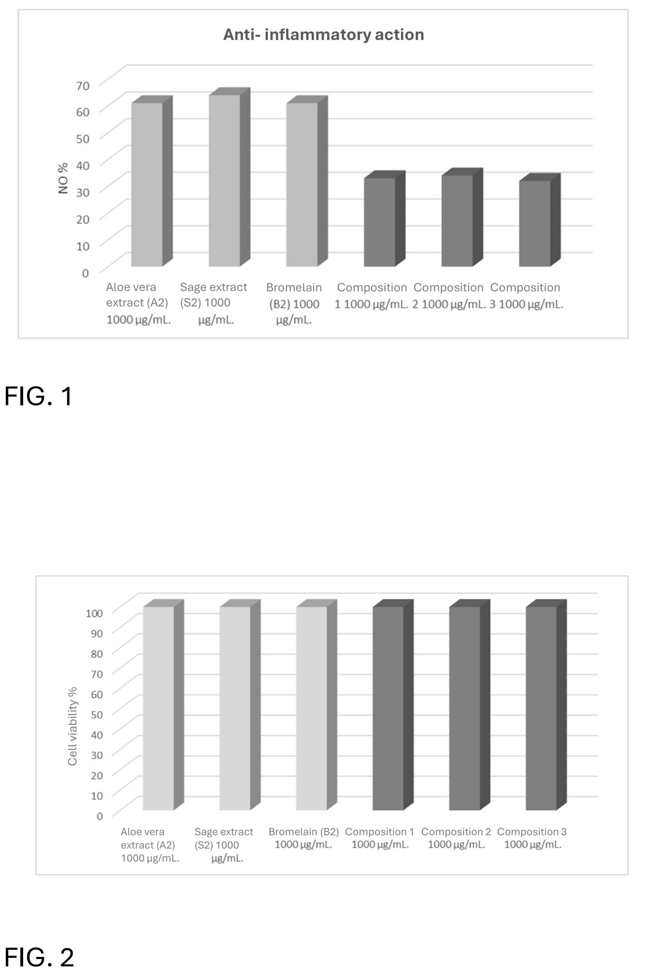

The data were reported as percentage of NO compared to the treatment with LPS only (1 μg/mL). The greater effectiveness of the composition according to the invention, compared to the components taken individually, in reducing NO is evident (Figure 1).

The MTT assay was performed under the same experimental conditions to verify the effect on the viability of the RAW 264.7 cells. The data were reported in Figure 2 as percentage of cell viability compared to the treatment with LPS only

(1 μg/mL). None of the extracts taken individually, nor the composition according to the present invention, impact cell viability. This data is indicative of the fact that the composition according to the invention is not toxic to cells.

### Example 7: Evaluation of the efficacy on in vitro-reconstructed human esophageal epithelium

This test is intended to evaluate the ability of the composition according to the invention to exert a barrier and regenerative effect against the human esophageal epithelium after acid irritation. In this regard, the following tests were carried out:

    i. morphological evaluation
    ii. evaluation of the barrier effect
    iii. evaluation of the expression of IL-6, classified as a cytokine with both pro- and anti-inflammatory action.

i. Morphological evaluation:

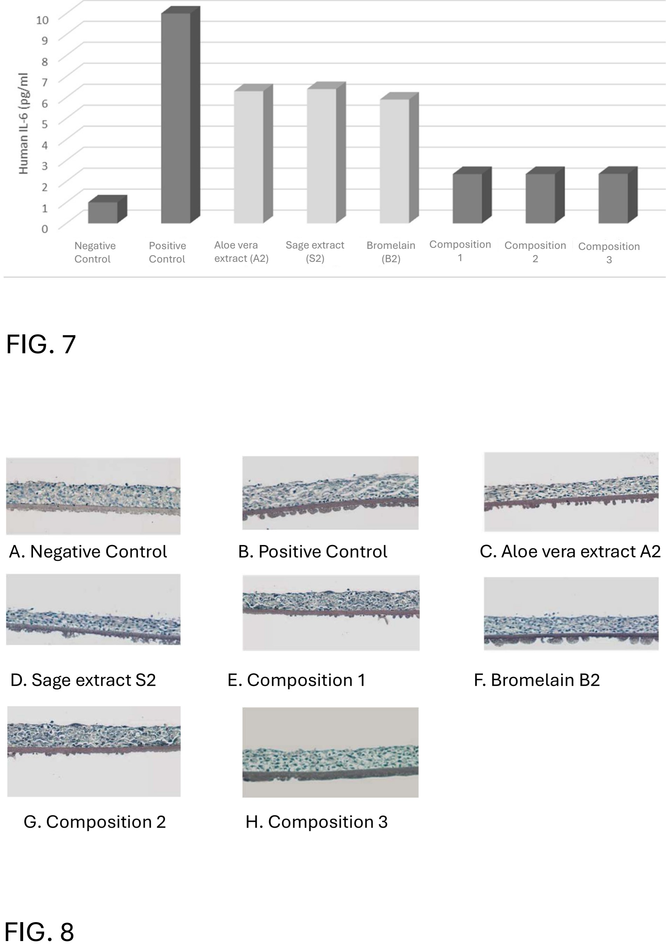

The assay is based on the morphological and structural evaluation of tissue inserts of in vitro-reconstructed human esophageal epithelium (HO2E/S/5 Reconstructed Human Oesophageal, EpiSkin®) after irritant insult induced by an acidic solution of 10% HCl, pH=1.1. The epithelium is inserted into a cell culture support comprising a plastic plate with 24 wells. The concentration of the acid solution is such as to simulate the conditions of an irritative state attributable to a clinical picture similar to gastroesophageal reflux; therefore the 3D epithelium models were first treated with a 10% HCl solution, pH=1.1, for 1', and after washing in PBS, they were treated with 60 μl of sample for 24h, in order to evaluate the regenerative capacity of the tested products.
After incubation, each of the cultures was fixed in a 4% formalin solution for histological analysis. Morphological analyses of the Reconstructed Human Oesophageal, EpiSkin®, were conducted by Hematoxylin/Eosin staining assay. The products under examination were compared with a negative control, consisting of a saline solution (0.9% NaCl) that has no action on the tissues, and a positive control, consisting of the acid solution.
Exemplary images of the morphological observations made on the inserts treated for 24 hours with the test samples after an acid insult of 1 minute are reported in Fig. 8. The effect has been quantified and the results are in Table 4 below.

| Legend | Absent 0 - | Mild < 10% + | Moderate ≥ 10 % - < 40% ++ | Severe < 40% +++ |
|---|---|---|---|---|
| | Negative | Positive | Aloe | Sage | Bromelain | Formulation |

| | control | control | vera extract | extract | | 1 |
|---|---|---|---|---|---|---|
| Cell Degeneration | - | +++ | ++ | ++ | ++ | + |
| Erosion | - | ++ | ++ | ++ | ++ | + |
| Necrosis | - | ++ | + | ++ | ++ | + |

Table 4

The histological analyses have shown a good regenerating capacity of the synergistic composition with respect to the individual components in terms of reduction of cell degeneration, epithelial erosion as well as necrosis after acid insult at 1'. The effect of the composition according to the invention is better than the one observed with the components thereof evaluated individually.

ii. Evaluation of the Barrier effect

The test aims to evaluate the possible barrier effect exerted by the test sample following damage induced by an irritant agent on in vitro-reconstructed human esophageal epithelium according to validated protocols.

The film-forming capacity was studied on an in vitro-reconstructed human esophageal epithelium model (*Reconstructed Human Oesophageal*) in order to evaluate their potential barrier effect.

The film-forming properties were determined by a multiparametric approach in the evaluation, which includes the transepithelial electrical resistance (TEER) measurement and determination of tissue permeability, the lucifer yellow assay.

An aliquot of 60 μl of each sample was topically applied to the surface of the epithelium 1h before the acid insult with a 10% HCl solution, pH 1.1, for 15' and 45', compared to a negative control, consisting of a saline solution (0.9% NaCl) that has no action on the tissues, and a positive control, consisting of the acid solution.

Measuring the transepithelial electrical resistance is a technique that is used to measure membrane integrity. Before the start of the experiment, TEER was measured by Millicell-ERS instrument (range 0-20 kU).

The determination of the TEER was carried out in the first measurement at time zero T0 before treatment with the test samples and at the end of the experimental design, T1.

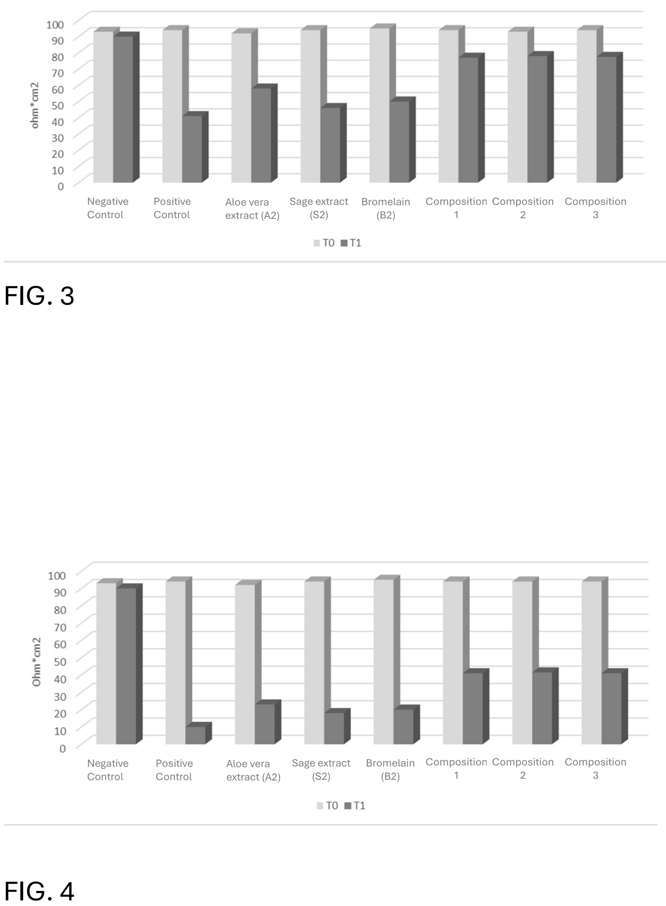

The results reported in Fig. 3 and 4, obtained after acid insult of 15 minutes and 45 minutes, respectively, demonstrate a greater transepithelial resistance, of the inserts treated with the composition according to the present invention, Formulation 1, 2 and 3, compared to the individual components.

Following TEER determination, the test sample excess was removed by washing and tissue permeability was determined by Lucifer Yellow assay.

Lucifer Yellow is a fluorescent dye used for tissue permeability studies. When the membranes are intact, this dye has a poor permeability, while in case of damaged structures the Lucifer Yellow shows a high permeability. Therefore, this test is used in order to determine the integrity of the membranes in the presence of substances to be examined.

For this purpose, 0.5 mL of Lucifer Yellow was placed in the apical compartment (AL), while 1 mL of saline was placed inside the basolateral compartment (BL). After an incubation period of 1 h at 37 °C, the passage of the dye Lucifer Yellow was determined by spectrofluorimetric analysis (Synergy H1 - Hybrid Reader - BioTek) at 428 nm (excitation) and at 535 nm (emission). The Flow was calculated by means of the following formula:

$$LY\ Flux\ \% = (RFU\ BL\ /\ RFU\ AP\ t{=}0)\ x\ 100$$

Where:
RFU= fluorescence measurement

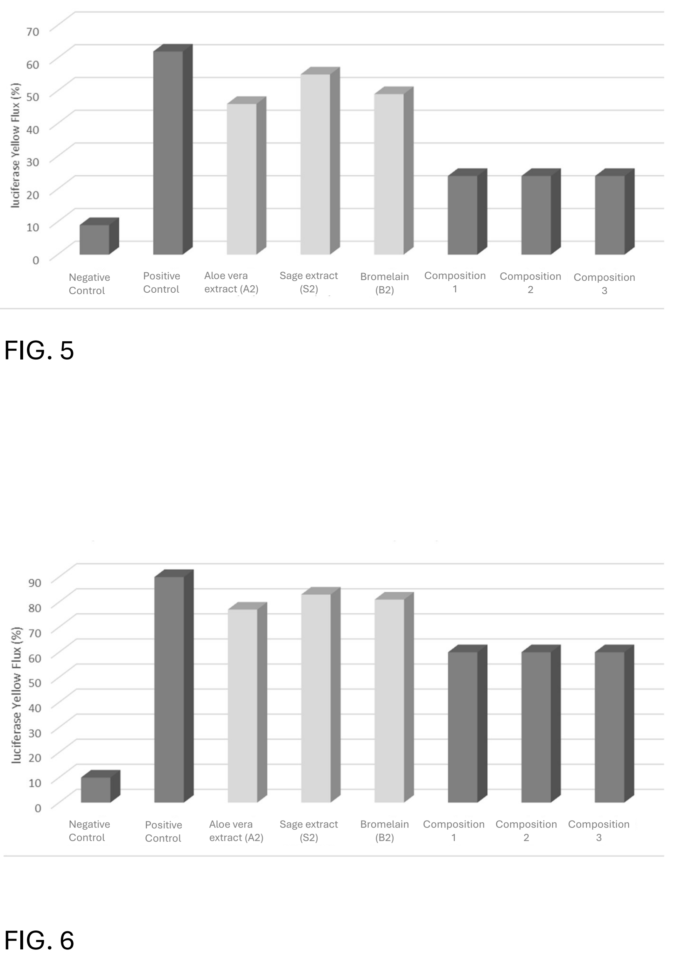

The results, reported in Figs. 5 and 6, obtained after 15 minutes and 45 minutes of acid insult, respectively, clearly show that, similarly to what was observed with the TEER assay, the composition according to the present invention is able to maintain membrane integrity, more markedly than the individual components tested.

iii. Evaluation of IL-6 expression

For the quantification of IL-6 production, an ELISA test was carried out.

The immunological techniques are based on the specificity that antibodies possess in recognizing and binding to the target protein (antigen) and offer several advantages over commonly used techniques for protein analysis.

ELISA protocols were developed with an indirect approach for the identification of the IL-6 expression levels in the culture medium of the reconstructed human esophageal epithelium inserts treated with the test compound.

The ELISA test is fast and versatile because it is applicable to a wide variety of scientific disciplines. In particular, it offers several advantages for the study of protein binders: the analysis is extremely sensitive to small amounts of antigen and micrometre-sized samples (<1mg by weight) are sufficient. The high sensitivity of the method is due to the fact that a single enzyme is able to catalyze the reaction of thousands of substrate molecules, thus amplifying the signal.

The human esophageal epithelium inserts were cultured in the following culture medium: Maintenance medium and incubated at 37 °C in an atmosphere of 5% $CO_2$ and 95% air. The experiments were conducted in 24-well multiwell.

To demonstrate the regenerating and anti-inflammatory capacity of the test samples on the in vitro-reconstructed human esophageal epithelium, after acid insult with a 10% HCl solution, pH= 1.1, for 1', ELISA assays were conducted in order to determine the concentration of IL-6 in the culture media of the tissue inserts, testing an aliquot of 100 μL of sample.

The results reported in Fig. 7, show a marked reduction of the concentration of IL-6 in the culture media of the inserts treated with the composition according to the invention, compared to those treated with the individual components, after acid insult of 1'.

**Example 8: Wound healing action- wound healing assay**

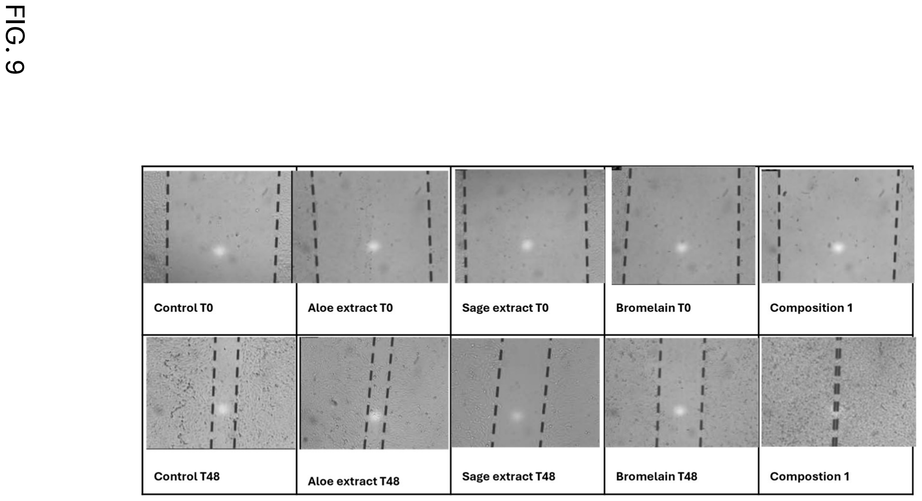

The scratch assay (or "wound healing assay") was performed on the MCF-10A breast epithelium line. The samples were used at a concentration of 25 μg/mL, chosen on the basis of the previous cell viability experiments.
The scratch assay is a simple method to study cell migration in vitro. This method is based on the observation of the behaviour of the cells following the creation of an artificial gap, called precisely a scratch. On a confluent monolayer of cells, this gap was created and the ability of the cells, at the edges of the newly created gap, to migrate in order to try and close the scratch, establishing new cell-cell contacts was evaluated.
The images were captured by microscope immediately after the creation of the gap (T 0h) and, after treatment with the test samples, at regular intervals of 48h, to then compare them and determine the rate of cell migration.
Immediately after the creation of the gap, the samples were treated to evaluate whether they were able to favour cell proliferation and, therefore, the closure of the scratch.
As can be evaluated from Figure 9, after 48h in the control the gap remains partially open, while in the MCF-10A treated with the composition according to the invention, the gap closes completely. Therefore, after 48h the composition according to the invention favoured the total healing of the artificially created wound. The individual components have only a partial effect on wound healing.

**Claims**

1. A composition comprising Aloe vera, Bromelain and Sage for use in the treatment of mucosal lesions of the esophageal tract and/or for the treatment of symptoms of gastroesophageal reflux.

2. The composition for use according to claim 1, wherein said composition comprises, per 100 g of finished product:

   60-80% Aloe vera extract;
   15-25% Sage extract;
   0.5 - 2% bromelain.

3. The composition for use according to claim 1, wherein said composition comprises, per 100 g of finished product:

   70% Aloe vera extract;
   20% Sage extract;
   1% bromelain.

4. The composition for use according to one of the claims from 1 to 3, wherein said Aloe vera extract is extracted from whole leaves.

5. The composition for use according to one of the claims from 1 to 4, wherein said Sage extract is extracted from leaves.

6. The composition for use according to one of the claims from 1 to 5, which also comprises food or pharmaceutical grade excipients, selected from the group comprising: diluents, adsorbents, lubricants, glidants, colorants, surfactants, antioxidants, sweeteners, flavorings, binders, disaggregants, plasticizers, viscosifiers, emulsifiers, humectants, wetting agents, preservatives, chelators and the like.

7. The composition for use according to one of the claims from 1 to 6, which is formulated in liquid form.

8. A supplement comprising Aloe vera, Bromelain and Sage for use in the prevention and improvement of mucosal lesions of the esophageal tract and/or in the prevention and improvement of symptoms of gastroesophageal reflux.

FIG. 1

FIG. 2

FIG. 3

FIG. 4

FIG. 5

FIG. 6

FIG. 7

A. Negative Control

B. Positive Control

C. Aloe vera extract A2

D. Sage extract S2

E. Composition 1

F. Bromelain B2

G. Composition 2

H. Composition 3

FIG. 8

FIG. 9

Europäisches
Patentamt

European
Patent Office

Office européen
des brevets

## EUROPEAN SEARCH REPORT

**Application Number**

EP 25 21 7061

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 101 537 156 A (YINGYING CAO) 23 September 2009 (2009-09-23) * Summary; claims 1-3 * | 1-8 | INV. A61K36/537 A61K36/886 A61K38/48 A61P1/04 |
| A | US 9 603 871 B2 (LIVELEAF INC [US]) 28 March 2017 (2017-03-28) * paragraph [0044] * * paragraph [0048] * * paragraph [0085] * | 1-8 | |
| A | US 6 291 533 B1 (FLEISCHNER ALBERT M [US]) 18 September 2001 (2001-09-18) * paragraph [0001] * | 1-8 | |
| A | IT LI20 060 004 A1 (LLOYDS INTERNAT CREDIT LLC) 23 August 2007 (2007-08-23) * paragraph [0121] * | 1-8 | |
| A | JP 2009 096728 A (TAIYO KAGAKU KK) 7 May 2009 (2009-05-07) * paragraph [0015] * * paragraph [0019] * | 1-8 | **TECHNICAL FIELDS SEARCHED (IPC)** A61K A61P |
| A | IT 2021 0001 1879 A1 (AURORA BIOFARMA S R L [IT]) 10 November 2022 (2022-11-10) * summary; paragraph [0108]; examples 4-6 * | 1-8 | |
| A | CN 109 730 222 A (SHENZHEN CHUNRUI TRADE CO LTD) 10 May 2019 (2019-05-10) * claim 4 * | 1-8 | |

The present search report has been drawn up for all claims

| Place of search | Date of completion of the search | Examiner |
|---|---|---|
| Munich | 12 March 2026 | Markopoulos, Eytyxia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 25 21 7061

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2026

| Patent document cited in search report | | Publication date | Patent family member(s) | | Publication date |
|---|---|---|---|---|---|
| CN 101537156 | A | 23-09-2009 | NONE | | |
| US 9603871 | B2 | 28-03-2017 | EP | 2934511 A2 | 28-10-2015 |
| | | | HK | 1216393 A1 | 11-11-2016 |
| | | | MX | 376916 B | 07-03-2025 |
| | | | TW | 201438715 A | 16-10-2014 |
| | | | US | 8716351 B1 | 06-05-2014 |
| | | | US | 8716352 B1 | 06-05-2014 |
| | | | US | 8716353 B1 | 06-05-2014 |
| | | | US | 8772352 B1 | 08-07-2014 |
| | | | US | 8809399 B1 | 19-08-2014 |
| | | | US | 8822545 B1 | 02-09-2014 |
| | | | US | 2014178493 A1 | 26-06-2014 |
| | | | US | 2014178494 A1 | 26-06-2014 |
| | | | US | 2014178495 A1 | 26-06-2014 |
| | | | US | 2014205678 A1 | 24-07-2014 |
| | | | US | 2014370118 A1 | 18-12-2014 |
| | | | US | 2015010645 A1 | 08-01-2015 |
| | | | US | 2015110895 A1 | 23-04-2015 |
| | | | US | 2015190449 A1 | 09-07-2015 |
| | | | US | 2016113964 A1 | 28-04-2016 |
| | | | US | 2016310455 A1 | 27-10-2016 |
| | | | US | 2017007643 A1 | 12-01-2017 |
| | | | US | 2018000862 A1 | 04-01-2018 |
| | | | US | 2018140633 A1 | 24-05-2018 |
| | | | US | 2018318345 A1 | 08-11-2018 |
| | | | WO | 2014099686 A2 | 26-06-2014 |
| US 6291533 | B1 | 18-09-2001 | US | 6291533 B1 | 18-09-2001 |
| | | | US | 6503529 B1 | 07-01-2003 |
| | | | US | 2003044473 A1 | 06-03-2003 |
| IT LI20060004 | A1 | 23-08-2007 | ------------------------------ | | |
| JP 2009096728 | A | 07-05-2009 | NONE | | |
| IT 202100011879 | A1 | 10-11-2022 | ------------------------------ | | |
| CN 109730222 | A | 10-05-2019 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011038945 A
- US 2009208588 A

- IT 201800004332 A1

**Non-patent literature cited in the description**

- **BASIM AL-SULIVANY et al.** Pathology And Medicinal Plant Treatment Of Gastroesophageal Reflux Disease. *African J Gastroenterology and Hepatology*, 2024, vol. 7